# EUROPEAN PATENT SPECIFICATION

(11) **EP 1 776 143 B1**
(45) Date of publication and mention of the grant of the patent: **02.01.2008**
(21) Application number: 05771854.6
(22) Date of filing: 21.06.2005
(51) Int. Cl.: A61K 47/48, C08G 65/329

(54) **BIFUNCTIONAL DERIVATIVES OF POLYETHYLENE GLYCOL, THEIR PREPARATION AND USE**
BIFUNKTIONALE DERIVATE VON POLYETHYLENGLYKOL, IHRE HERSTELLUNG UND VERWENDUNG
DERIVES BIFONCTIONNELS DE POLYETHYLENEGLYCOL: PREPARATION ET UTILISATION

(30) Priority: 21.06.2004 IT PD20040159
(43) Date of publication of application: 25.04.2007
(73) Proprietor: Universita'Degli Studi di Trieste, I-34127 Trieste (IT)
(72) Inventor: BONORA, Gian Maria, I-35126 Padova (IT); CAMPANER, Pietro, I-33100 Udine (IT); DRIOLI, Sara, I- 34123 Trieste (IT)
(74) Representative: Gervasi, Gemma
(86) International application number: PCT/EP2005/052876
(87) International publication number: WO 2005/123139

(56) References cited:
- WO-A-03/031066
- WO-A-20/04074345
- ROBERTS M J ET AL: "Chemistry for peptide and protein PEGylation" ADVANCED DRUG DELIVERY REVIEWS, AMSTERDAM, NL, vol. 54, no. 4, 17 June 2002 (2002-06-17), pages 459-476, XP002293146 ISSN: 0169-409X
- LI JING ET AL: "Synthesis of polyethylene glycol (PEG) derivatives and PEGylated-peptide biopolymer conjugates" BIOMACROMOLECULES, ACS, WASHINGTON, DC, US, vol. 4, no. 4, 17 May 2003 (2003-05-17), pages 1055-1067, XP002328259 ISSN: 1525-7797

## Description

### FIELD OF THE INVENTION

The invention relates to derivatives of polyethylene glycol of different molecular weights homo- or hetero-bifunctionalized at the hydroxyl end groups with reactive bifunctional molecules (biPEG), to their synthesis process and to their use as biocompatible carriers and/or stabilizers by means of the conjugation thereto of substances used in the diagnostic, prophylactic or therapeutic field, or reagents and catalysts used for the synthesis of supported organic syntheses.

### STATE OF THE ART

Polyethylene glycol (PEG) and polymer materials based thereon are gaining considerable interest for their biotechnological and pharmaceutical applications *(*R.B. Greenwald, Y.H Choe, J. McGuire, C.D. Conover, Adv. Drug Delivery Rev., 5, 217-250, 2003*).* The PEGylation of peptides and proteins constitute the main example of such applications *(*F.M. Veronese, Biomaterials, 22, 405 - 417, 2001*):* this process is able to expand therapeutic potential in that the molecule bound to PEG, while maintaining its original biological functions, demonstrates an increased stability to degradation processes occurring *in vivo.* In this respect the presence of these PEG chains, especially if of high molecular weight, can mask its protein surface and so prevent being attacked by compounds with degrading or blocking action, such as enzymes or antibodies or even by cells designated to destroy them. Furthermore, the resulting increased molecular size reduces its renal filtration thus maintaining the active PEG-conjugated molecules in the blood system for a longer period of time, hence advantageously modifying its pharmacokinetic properties. Finally PEG confers its own chemico-physical characteristics to the molecules bound thereto, improving their bioavailability and solubility and, in general, making them easier to administer *(*M.L. Nucci, R. Shorr, A. Abuchowski, Adv. Drug Delivery Rev., 6, 133 - 151, 1991).

Recently PEGylation of oligonucleotide chains, behaving as antisense and antigene agents *(*G.M. Bonora et al., Farmaco, 53, 634-637, 1998) or as ribozymes for the specific hydrolysis of RNA chains (L. Gold, J. Biol. Chem.,270, 13589-93584, 1995), have been investigated. In this case the charged polar chains of the oligonucleotides generally exhibit limited cell penetrability which can be increased by the amphiphilic character of the PEG chains. Also, an increased stability to enzyme degradation is again noted *(*G.M. Bonora et al., Bioconjugate Chem., 8, 793-797, 1997) as well as a prolonged circulation time in the blood stream, with an effect that increases with increasing polymer molecular weight.

Together with these examples, numerous small molecules have been used in the form of PEG-conjugates, again with the purpose of improving their pharmacological administration characteristics. Among these molecules, antitumour drugs such as doxorubicin and taxol, antimalarials such as artemisinin and various enzymatic inhibitors can be mentioned.

Recently, mixed conjugates such as peptides and oligonucleotides linked together in the same molecule, have received particular attention, as they can be characterised by a greater and more specifically addressed cellular accumulation *(*R. Eritja, A. Pons, M. Excarceller, E. Giralt, F. Albericio, Tetrahedron, 47, 4113-4120, 1991). The synthesis of said conjugates, as of those carrying other useful organic molecules, is of great interest because of the clear pharmacological advantages. A recent example used a heterofunctional PEG for coupling the protein FGF2 to the surface of an adenovirus to generate a new vector for use in gene therapy and for the purpose of minimizing its toxicity and expression in non target tissues and of exploiting the receptor specificity of said protein *(*J. Lanciotti et al., Mol. Ther., 8, 99-107, 2003*).*

For this purpose, if the carrier and stabilizer properties of PEG are to be utilised, reactive polymer derivatives must be available having end groups which can be effectively derivatized at different times and in different manners. However, selective chemical modification of a single end group is a difficult synthetic undertaking, given that a mixture of difficultly separable bi-, mono- and unprotected derivatives is inevitably obtained, especially if involving high molecular weight polydisperse polymer molecules, as indeed is PEG.

With regard to these polymers, a procedure of possible interest was formerly proposed using a commercial low molecular weight diamino PEG, being therefore of less applicative interest as a carrier and stabilizing agent *(*G.R. Ehteshami, S.D. Sharma, J. Porath, R.Z. Guzman, Reactive and Functional Polymers, 35, 135-143, 1997*).*

For these reasons a synthesis process was recently proposed aimed at producing commercial dihydroxy PEGs terminally protected by orthogonal groups therefore removable by different manners and times *(*S. Drioli, F. Benedetti, G.M Bonora, Reactive and Functional Polymers, 48, 119-128, 2001*).* With the aim of isolating and purifying the mono-protected polymer derivative, a subsequently removable ionisable carboxylic group was temporarily introduced. Purifying the mono-protected intermediate by efficient chromatographic procedures provides the required derivative for its subsequent final selective modification.

The difficulty of this procedure, however, has prompted the application of mono-protection procedures to amino PEG derivatives, already possessing an ionisable group at the terminal; said derivatives also offer better reactivity on the subsequent introduction of pharmacologically active molecules. On the basis of these factors a synthesis process was used which directly uses commercial dihydroxy PEG, it being available in a wide range of sizes and at a generally much lower cost than the diamino PEG previously employed.

In many of the methods known heretofore it has been possible to insert at the end groups of commercial PEGs, simple organic molecules carrying for example an amino terminal group, by activating the polymer hydroxyl groups. As reported in the literature, conjugation of a new molecule to PEG therefore takes place via derivatization of the polymer terminal hydroxyl groups (M.J. Roberts, M.D. Bentley, J.M. Harris, Adv. Drug Delivery Rev. 54, 459-476,2002). However, should base-labile groups be present at this stage of the synthesis, for example where it is necessary to conjugate a protected oligonucleotide sequence with said groups *(*G.M. Bonora, M. Ballico, P. Campaner, S. Drioli, I. Adamo, Nucleosides, Nulcleotides & Nucleic Acids,. 22, 1255-125, 2003*),* the introduction of molecules bearing basic amino groups can cause partial deprotections and consequent synthesis difficulties, in addition to possible detachment of molecules conjugated to the support by base-labile bonds.

From this observation the need has arisen for the development of a new synthesis strategy which comprises the activation not of the functional group or groups of PEG but of the functional group or groups of a polyfunctional molecule required for modifying the PEG itself, in order to avoid the direct reaction of hydroxyl groups, which are activated and/or protected by base-labile groups, with basic amines. The ultimate aim is to obtain a new PEG derivative which can be easily prepared from commercial dihydroxy PEG, and therefore also polydispersed, and which can be used for carrying and/or stabilizing molecules, being the same or different one from the other, by their conjugation on the same polymer support.

An object of the present invention is therefore to obtain new homo- or hetero-bifunctional derivatives of polyethylene glycol (PEG), namely derivatives characterised by equally or differently reactive terminal chemical functionalities.

Another object of the present invention is to obtain new homo- or hetero-bifunctional derivatives of polyethylene glycol (PEG) by a simplified synthesis process of easy industrial applicability.

Another object of the present invention is to obtain new homo- or hetero-bifunctional derivatives of polyethylene glycol (PEG) suitable for the conjugation of compounds usable for diagnostic, prophylactic or therapeutic purposes or reagents or catalysts usable in supported synthesis processes.

### SUMMARY

For the above-mentioned objects, the inventors have identified in the new homo- or hetero-bifunctional derivatives of polyethylene glycol (PEG) characterised by equally or differently reactive terminal chemical functionalities, the solution for obtaining bifunctional PEG derivatives that are easily synthesizable and easily conjugatable with compounds of different kind starting from commercial dihydroxy PEGs of various molecular weights.

The invention therefore provides bifunctional polyethylene glycol (PEG) derivatives of general formula (I)

P'HN-(H₂C)_{n'}-HN-CO-O-PEG-O-CO-NH-(CH₂)ₙ-NHP (I)

in which:
the polyethylene glycol has a molecular weight between 2,000 and 40,000,
P, P' can be the same or different one from the other and be a hydrogen or a
protecting group for the amino group,
n and n', being the same or different one from the other, are between 1 and 6.

A further aspect of the invention is the synthesis process for the homo- and hetero-bifunctional derivatives of polyethylene glycol (PEG) of general formula (I)
characterised by the following steps:
- derivatization of a first terminal hydroxyl group of the polyethylene glycol with a bifunctional compound activated at one functional group and optionally mono-protected at the second functional group,
- derivatization of the second hydroxyl group of the polyethylene glycol with a bifunctional compound being the same or different from the previous one,
- ionisation of the free functional group of the bifunctional PEG derivative obtained,
- purification of the ionic bifunctional PEG derivative obtained,
- neutralization of the ionised functional group of the purified ionic bifunctional PEG derivative and its optional protection.

A further aspect of the invention is the use of homo- or hetero-bifunctional polyethylene glycol (PEG) derivatives of general formula (I) for the preparation of compounds by conjugation with compounds of differnt kind, being said compounds the same or different one from the other, usable for diagnostic, prophylactic and therapeutic purposes or for supported synthesis processes.

### BRIEF DESCRIPTION OF THE FIGURES

Figure 1: IR spectrum of the compound Z-NH-(CH₂)₃-isocyanate of example 1.b).
Figure 2: ¹H-NMR spectrum of the compound Z-NH-(CH₂)₃-isocyanate of example 1.b).
Figure 3: ¹³C-NMR spectrum of the compound Z-NH-(CH₂)₃-isocyanate of example 1.b).
Figure 4: ¹H-NMR spectrum of the compound BOC-NH-CO-O-PEG-O-CO-NH-Z of example 2 in CDCl₃
Figure 5: ¹H-NMR spectrum of the compound BOC-NH-CO-O-PEG-O-CO-NH₂ of example 3 in CDCl₃
Figure 6: ¹H-NMR spectrum of the compound Z-NH-CO-O-PEG₆₀₀₀-NH₂ in DMSO-d⁶
Figure 7: : ¹H-NMR spectrum of the compound Z-NH-CO-O-PEG₆₀₀₀-NH-Gly-Phe-COOMe in DMSO-d⁶
Figure 8: : ¹H-NMR spectrum of the compound MeOOC-Phe-Val-NH-CO-O-PEG₆₀₀₀-NH-CO-NH-Gly-Phe-COOMe in DMSO-d⁶

### DETAILED DESCRIPTION OF THE INVENTION

These and other aspects of the present invention together with the characteristics and advantages will be better understood from the following detailed description which describes, by way of non-limiting examples of the invention, the synthesis of bifunctional PEG derivatives, in which the terminal hydroxyl groups thereof have been derivatized with bifunctional compounds, having the significance of bifunctional linkers for the subsequent conjugation between PEG and the molecules to be carried and/or stabilized, and their structural characterisation.

The homo- and hetero-bifunctional derivatives of polyethylene glycol (PEG) of general formula (I),

P'HN-(H₂C)ₙ,-HN-CO-O-PEG-O-CO-NH-(CH₂)ₙ-NHP (I)

in which:
the polyethylene glycol has a molecular weight of between 2,000 and 40,000, P, P' can be the same or different one from the other and be a hydrogen or a protecting group for the amino group,
n and n', being the same or different one from the other, are between 1 and 6, are prepared starting from a commercial dihydroxy PEG, available at low cost and in a wide range of molecular weights.

When P and P' groups are different from hydrogen, and are therefore a group protective of the amino group, they are protecting groups known to the expert of the art (for reference see Protective Groups in Organic Synthesis, T.W. Greene and P.G.M. Wuts Ed., J. Wiley & Sons, New York, 1999, III Edition, pp.503-614*)* and are carbamates, amides and groups specific for N-alkyl and N-arylamine, for imino derivatives, for enamino derivatives, derivatives with N-heteroatoms (for example N-metals, N-N derivatives, N-P derivatives, N-Si derivatives, N-sulfenyl derivatives and N-sulfonyl derivatives). Among these the preferred protecting groups are carbamates and amides and in particular for carbamates: carbamates in general, ethyl substituted carbamates, carbamates with assisted hydrolysis and photolytic hydrolysis, urea type, and for amides: amides in general, amides with assisted hydrolysis, cyclic imide derivatives.

For modifying the common commercial dihydroxy PEG of choice, the pre-selected procedure comprises the sequential introduction of bifunctional linkers which on the one hand enable polymer reactivity to be optimised and on the other hand enable it to be purified from all the unmodified or totally modified polydisperse polymer derivatives obtained by virtue of the ionisability of a generally amino terminal. Protection with groups that can be selectively introduced and removed enables the widest choice of synthesis methods relating to the conjugation of chemical species, even where they are different, on the same polymer support.

The synthesis process for the homo- or hetero-bifunctional polyethylene glycol (PEG) derivatives of general formula (I), according to the above-mentioned essential steps, can be represented by the following general scheme: 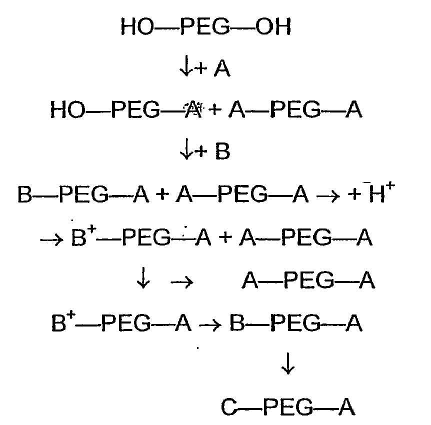 where:
A = -O-CO-NH-(CH₂)ₙ-NHP; B = -O-CO-NH-(CH₂)_{n'} -NH₂;
C = -O-CO-NH-(CH₂)_{n'} -NHP'.

The novel characteristic of the process is related to the ability to activate a functional group of the bifunctional compound chosen for derivatization of commercial dihydroxy PEG, having the significance of conjugation linker and not, as is normally done, of the hydroxyl group of the PEG to be derivatized. Moreover, the type of bifunctional compound or linker that is introduced provides at the same time an ionisable terminal which enables easy and selective purifications by means of suitable exchanging supports. Again, the chemical bond between PEG and said bifunctional compound or linker possesses predictably useful biodegradability characteristics, as has also the bond between the reactive terminal of the bifunctional compound or linker and the conjugated compound, for example a drug. The molecule used is of bifunctional type, generally a diamine, and for the first derivatization preferably exhibits a mono-protection, such as benzyl-oxycarbonyl (Z) thus avoiding the formation of dimers of the type HO-PEG-O-CO-NH-(CH₂)ₙ-NH-CO-O-PEG-OH. Naturally, other groups can be used in so far as their stability under the reaction conditions allows, such as tert-butyl-oxy carbonyl (Boc) and the like as indicated previously.

By way of non-limiting examples of the present invention the synthesis of a biPEG is given hereinafter, starting from a commercial PEG, with a molecular weight of around 6000, functionalized at the first hydroxyl group with the activated mono-protected diamine Z-NH-(CH₂)₃-isocyanate. Said linker is synthesised in accordance with the following scheme:

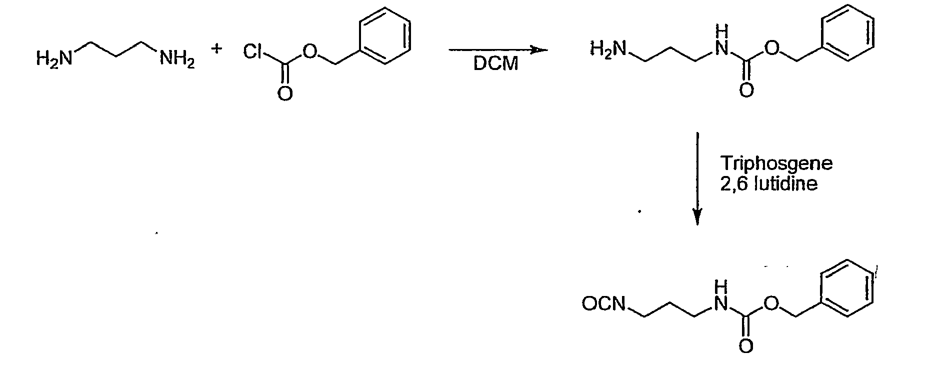

In detail the synthesis of Z-NH-(CH₂)₃-isocyanate is the following:

### Example 1: synthesis of Z-NH-(CH₂)₃-isocyanate

### 1.a) Synthesis of N-Z-1,3-diaminopropane

A solution containing 0.5 eq. of Z-Cl in 25 ml of CH₂Cl₂ is added drop-wise to a solution of 1 ml 1,3-diaminopropane in 60 ml of CH₂Cl₂ in an ice bath; the system is left under stirring in an ice-bath for 1 hour, monitoring progress of the reaction by thin layer chromatography (TLC) (iodine vapour test). At the end of the reaction, the solution is filtered to separate the white precipitate formed (diprotected amine) and extracted with a 0.1 N solution of HCl (monitoring the acidity of the aqueous phase by pH). After adjusting the pH of the aqueous phase to a value between 8.5 and 9, it is again extracted with CH₂Cl₂ for the monoprotected amine; the organic phase is then dried over anhydrous Na₂SO₄. After removing the CH₂Cl₂ by Rotavapor a yellow oil is obtained which, once re-dissolved with methyl t-butylether (MTBE) in an ice-bath, gives a white solid identified by ¹H-NMR and IR spectrophotometry as N-Z-1,3-diaminopropane.

### 1.b) Synthesis of Z-NH-(CH₂)₃-isocyanate

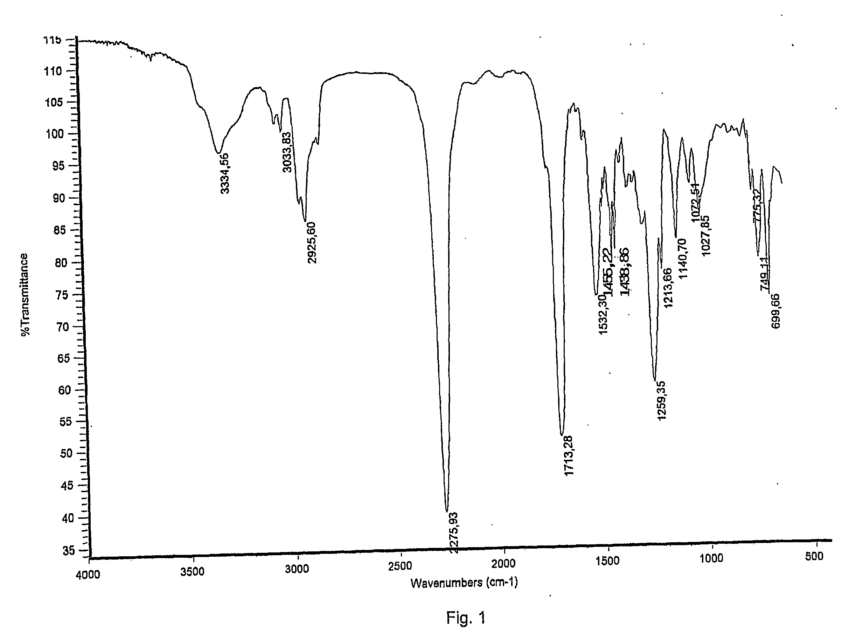

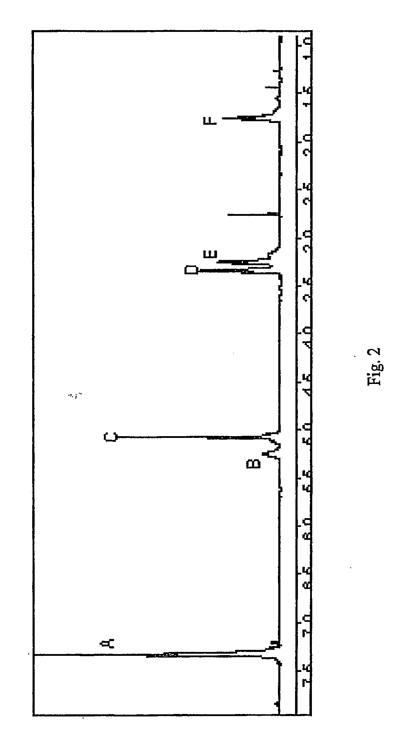

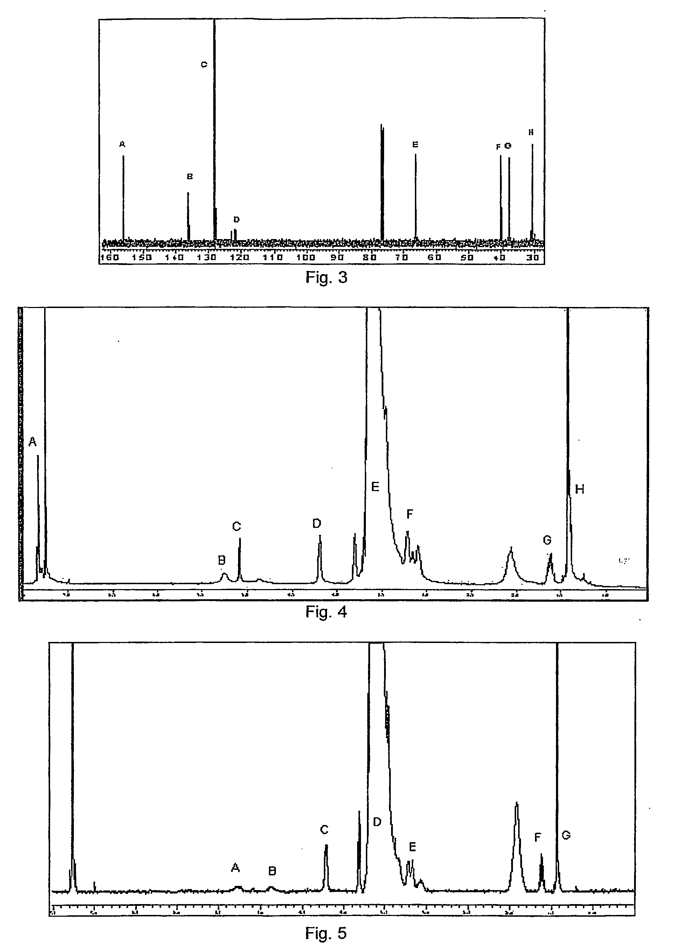

0.296 mg (1 mmol) of triphosgene are dissolved in 3 ml of CH₂Cl₂ and left under stirring under argon atmosphere; using a dropping funnel a solution consisting of 300 µl of 2,6-lutidine and 0.208 g (1 mmol) of N-Z-1,3-diaminopropane are added drop-wise over a period of 30 minutes. The system is left under stirring under argon atmosphere for 1 hour; the solvent is then removed by Rotavapor, to obtain a reddish oil from which, by adding MTBE, a solid precipitates identified as being the non-derivatized monoamine. The solid is removed then the mother liquors are dried to obtain a yellow-orange oil which, after characterisation with IR (Fig 1), ¹H-NMR (Fig. 2-table 1) and ¹³C-NMR (Fig. 3-table 2), was identified as being the desired Z-NH-(CH₂)₃-isocyanate (yield: 75%).

**Table 1: ¹H NMR analysis data of Z-NH-(CH₂)₃-isocyanate**

| | **Peaks found** | **ppm** | **Integral value calc.** | **Integral value found** |
|---|---|---|---|---|
| A | H aromatic Z | 7.4 | 5 | 5 |
| B | NH urethane | 5.45 | 1 | 0.7 |
| C | CH₂-Z | 5.1 | 2 | 2.3 |
| D | CH₂-NCO | 3.3 | 2 | 2.2 |
| E | CH₂-NH-CO | 2.8 | 2 | 1.9 |
| F | CONH-CH₂-CH₂-CH₂-NH₂ | 1.6 | 2 | 1.8 |

**Table 2: ¹³C NMR analysis data of Z-NH-(CH₂)₃-isocyanate (CDCl₃)**

| | **Peaks found** | **ppm** |
|---|---|---|
| A | CO urethane | 156.7 |
| B | Z-CH-CH₂ | 136.6 |
| C | Z | 128.6-128.1 |
| D | NCO | 122.1 |
| E | Z-CH₂-O | 66.8 |
| F | CH₂-NCO | 40.3 |
| G | CH₂-NH-CO | 38.2 |
| H | OCN-CH₂-CH₂-CH₂-NH-CO | 31.4 |

Starting from the linker obtained in example 1.b) and from a commercial PEG of about 6,000 molecular weight, the biPEG was synthesised in accordance with the following scheme and also described in detail in examples 2 and 3:

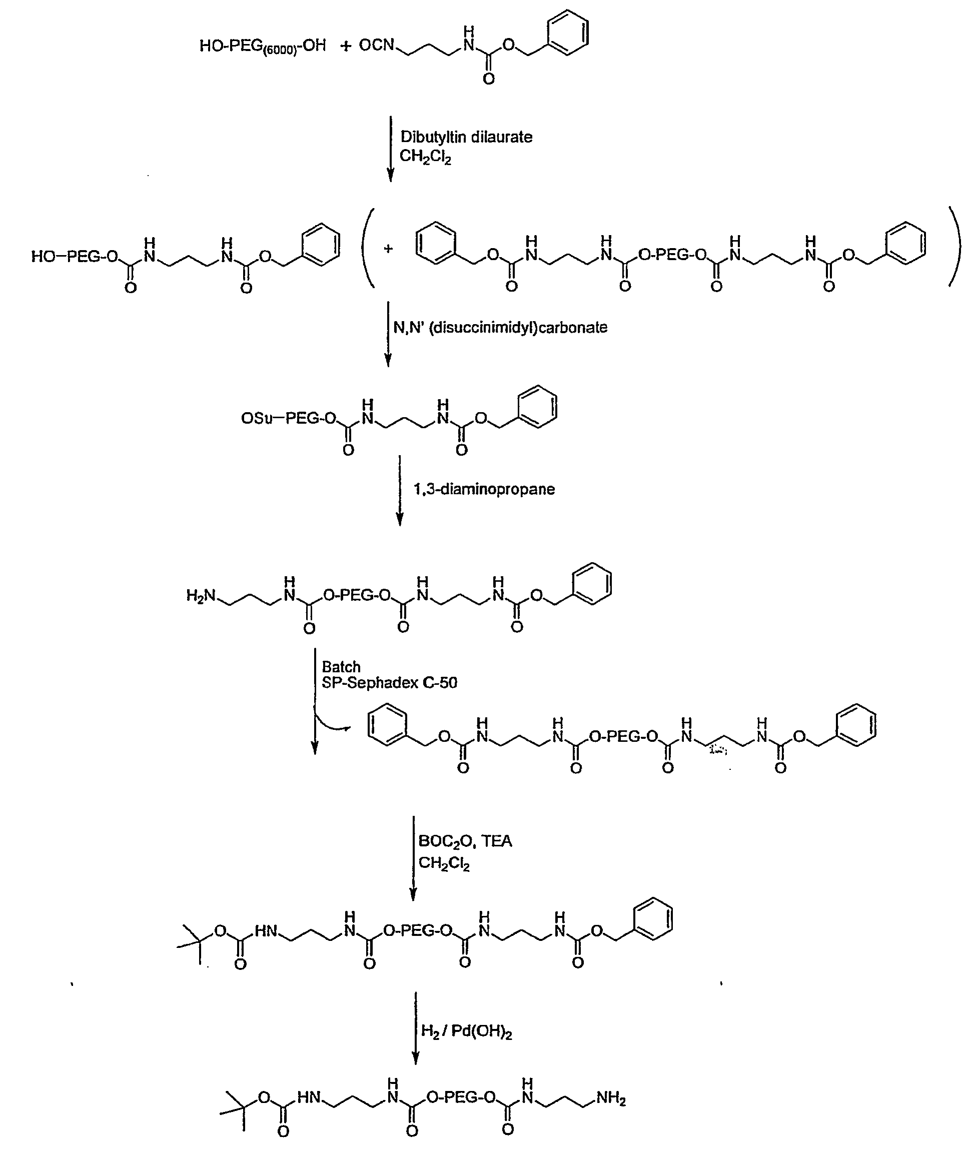

### Example 2: Synthesis of BOC-NH-CO-O-PEG₆₀₀₀-O-CO-NH-Z

### 2.a) Synthesis of Z-NH-CO-O-PEG-O-CO-NH₂

20 µl of the catalyst (Sn-dibutyl-dilaurate) are added to 1.0 g (0.167 mmol) of OH-PEG₆₀₀₀-OH, coevaporated with anhydrous CH₂Cl₂ (x 2), and left to dry using a rotary pump; the solution thus obtained is added drop-wise to a solution containing 2.5 eq. of Z-NH-(CH₂)₃-isocyanate in 3 ml of CH₂Cl₂. The system is left under stirring for 18 hours under argon atmosphere at ambient temperature. The product mixture is then precipitated by MTBE in an ice-bath, filtered through a Gooch 3G crucible, washed with ethyl ether (Et₂O) and recrystallized from ethyl alcohol (EtOH).

The PEG derivatives mixture (1 g, 0.167 mmol) is coevaporated with anhydrous CH₂Cl₂ (x 2) and left to dry using a rotary pump. After dissolving in a mixture of solvents (3.5 ml of CH₂Cl₂, 1 ml of AcCN and 0.5 ml of pyridine) the mixture is reacted with 4 eq. of N,N' (disuccinimidyl) carbonate (171 mg, 0.668 mmol). The system is left under stirring for 18 hours under argon atmosphere at ambient temperature. The product mixture is then precipitated by MTBE in an ice-bath, filtered through a Gooch 3G crucible, washed with Et₂O and recrystallized from EtOH.

The PEG derivatives mixture (1 g) is coevaporated with anhydrous CH₂Cl₂ (x 2) and left to dry using a rotary pump; the product is then dissolved in the minimum quantity of anhydrous CH₂Cl₂ and reacted with 3 eq. of 1,3-diaminopropane. The system is left under stirring for 18 hours under argon atmosphere at room temperature. The product mixture is then precipitated by MTBE in an ice-bath, filtered through a Gooch 3G crucible, washed with Et₂O and recrystallised from

EtOH.

### 2.b) Purification by column

The mixture of PEG derivatives (1.0 g) obtained is passed through a column (2.5 x 30 cm) packed with cationic exchange resin SP-Sephadex C50 equilibrated in milliQ H₂O. The fractions (10 ml) are collected using a flow equal to 1.40 ml/min.

The elution is continued until a UV/Vis absorbance at 260 nm is detected due to the presence of the Z group. The desired derivative is then recovered by eluting the column with a 0.1 M HCl solution in milliQ H₂O. The fractions containing H₂N-CO-O-PEG-O-CO-NH-Z, after combining, are evaporated under vacuum by Rotavapor at a temperature no higher than 35°C. The white solid thus obtained is dissolved in AcCN and then filtered off, so as to remove traces of any salts that are present. The organic phase is then dried over Na₂SO₄ and the solution is concentrated to remove part of the solvent by Rotavapor. The PEG derivative is precipitated in an ice-bath by adding MTBE, filtered through a Gooch 3G crucible, washed repeatedly with Et₂O and stored in a dryer over anhydrous KOH. The degree of product functionalization is evaluated by spectrophotometric analysis (TNBS test) and by ¹H-NMR analysis; a derivatization equal to 0.98 moles of -NH₂ per mole of Z-NH-CO-O-PEG-O-CO-NH₂ is found. From 1.00 g, 0.4 g of pure product are obtained.

### 2.c) Batch purification

40 ml of SP-Sephadex C50 resin already suspended in milliQ H₂O are left to come to equilibrium. 2.0 g of the product mixture dissolved in 10 ml of miliQ H₂O are added to the resin thus prepared. The system is left under moderate stirring for about ½ hour.

The resin is washed through a Gooch crucible with 150 ml of milliQ H₂O By evaporating the H₂O under vacuum by Rotavapor 1.25 g of the compound are obtained.

The resin is then treated with 90 ml of a 0.1 M HCl solution. After evaporating the H₂O by Rotavapor a white solid is obtained which is treated as described in the preceding paragraph to remove salts that are present. Following precipitation an overall 0.65 g of compound are obtained.

### 2.d) Synthesis of BOC-NH-CO-O-PEG-O-CO-NH-Z

100 mg (0.0157 mmol) of previously purified PEG derivative are coevaporated with anhydrous CH₂Cl₂ (x 2) and left to dry using a rotary pump. The PEG derivative is dissolved in the minimum quantity of anhydrous CH₂Cl₂; 12 µl of triethylamine (TEA) are added to the solution thus obtained. The system is maintained under stirring and 4 eq of BOC₂O (14 mg) dissolved in CH₂Cl₂ are added drop-wise. The system is left under stirring for 18 hours at room temperature. The product is then precipitated by MTBE in an ice-bath, filtered through a Gooch 3G crucible, washed with Et₂O and recrystallised from EtOH. The ¹H-NMR spectrum of the compound is given in fig. 3/tab. 3.

**Table 3: ¹H-NMR analysis data of BOC-NH-PEG-NH-Z (in CDCl₃)**

| | **Peaks found** | **ppm** | **Integral value calc.** | **Integral value found** |
|---|---|---|---|---|
| A | H aromatic Z | 7.4 | 5 | 5 |
| B | NH urethane | 5.2 | 2 | 1.3 |
| C | CH₂-Z | 5.1 | 2 | 1.8 |
| D | PEG-CH₂-O-linker | 4.2 | 4 | 3.5 |
| E | PEG | 3.8-3.2 | 454 | 454 |
| F | BOC-NH-CH₂-CH₂-CH₂-NH-PEG-NH-CH₂-CH₂-CH₂-NH-Z | 3.0-3.2 | 8 | 7 |
| G | BOC-NH-CH₂-CH₂-CH₂-NH-PEG-NH-CH₂-CH₂-CH₂-NH-Z | 1.6 | 4 | 3.5 |
| H | BOC | 1.4 | 9 | 8.7 |

### Example 3: Synthesis of BOC-NH-CO-O-PEG-O-CO-NH₂

20 mg of PEG derivative are dissolved in MeOH in a three-neck 50 ml flask and Pd(OH)₂ is added as catalyst in a quantity equal to 20% by weight on the substrate. The system is left under stirring overnight under H₂ atmosphere and room temperature.

At the reaction end, after removing the catalyst via filtration through a pleated filter, the MeOH is removed under reduced pressure; the solid obtained is re-dissolved in AcCN and then precipitated by MTBE in a ice-bath, filtered through a Gooch 3G crucible, washed with Et₂O and stored over KOH.

The ¹H-NMR spectrum of the compound is given in fig. 4/tab. 4.

**Table 4: ¹H-NMR analysis data of Z-NH-PEG₆₀₀₀-NH-CO-NH-Gly-Phe-COOMe (in DMSO)**

| | **Peaks found** | **ppm** | **Integral value calc.** | **Integral value found** |
|---|---|---|---|---|
| A | NH urethane | 5.2 | 1 | 0.6 |
| B | NH urethane | 5.2 | 1 | 0.5 |
| C | PEG-CH₂-O-linker | 4.2 | 4 | 3.5 |
| D | PEG | 3.8-3.2 | 454 | 454 |
| E | BOC-NH-CH₂-CH₂-CH₂-NH-PEG-NH-CH₂-CH₂-CH₂-NH₂ | 3.0-3.2 | 8 | 7 |
| F | BOC-NH-CH₂-CH₂-CH₂-NH-PEG | 1.6 | 2 | 1.9 |
| G | BOC | 1.4 | 9 | 8.7 |

The bifunctional PEG derivatives of the invention can be usefully employed for carrier and/or stabilizer applications by conjugation with biologically active molecules. For the purposes of non-limiting illustration, the following example presents the conjugation of a bifunctional PEG, obtained as previously described, with two different amino acids.

### Example 4: synthesis of MeCOO-Phe-Val-NH-CO-NH-PEG₆₀₀₀-NH-CO-NH-Gly-Phe-COOMe

The conjugated PEG synthesis scheme is summarized as follows:

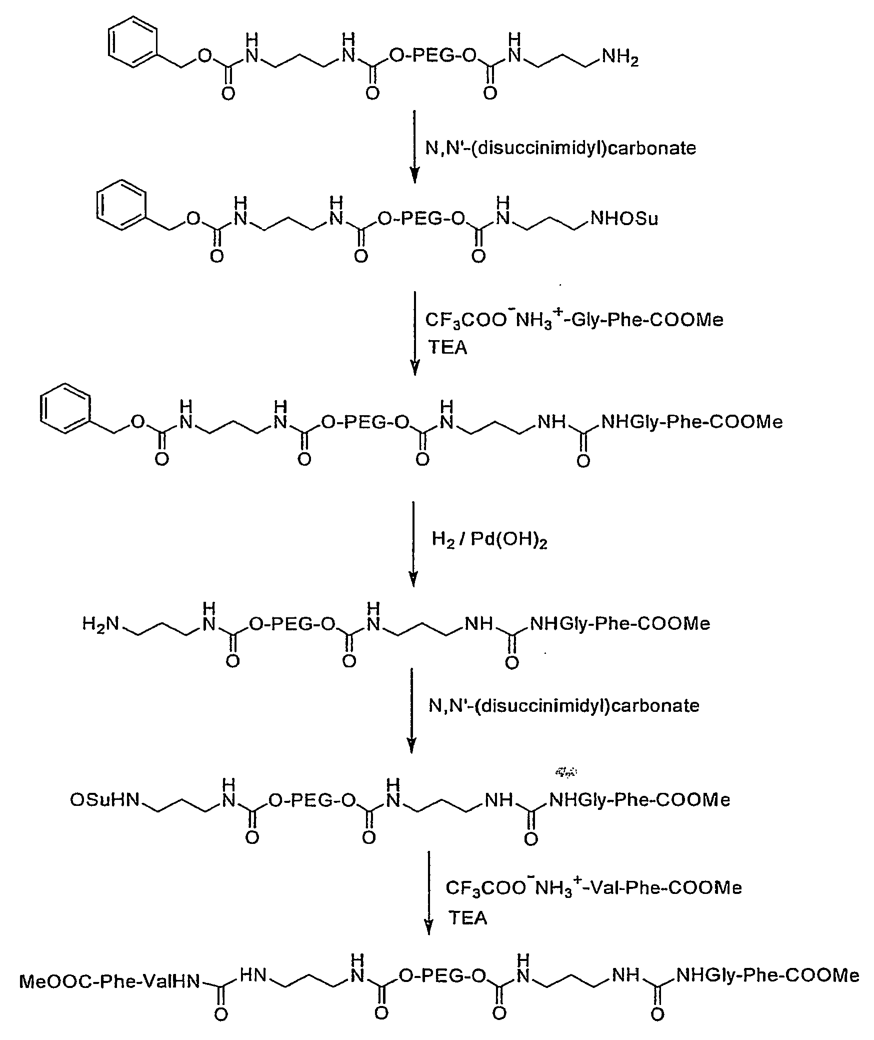

### 4.a) Synthesis of Z-NH-CO-O-PEG₆₀₀₀-NH-OSu

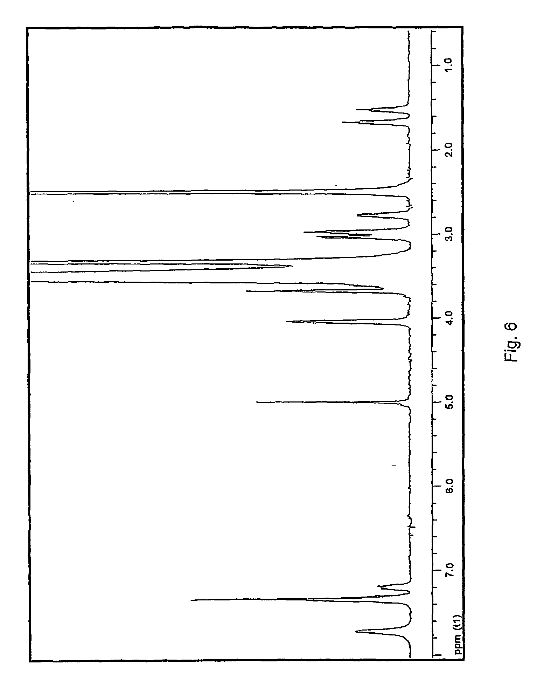

250 mg (0.039 mmol) of Z-NH-CO-O-PEG-NH₂ (Fig. 6 ¹H NMR spectrum /Tab. 5) are coevaporated with anhydrous CH₂Cl₂ (x 2) and left to dry using a rotary pump. After dissolving the PEG derivative in a mixture of solvents (1.5 ml of CH₂Cl₂ 0.5 ml of AcCN and 0.5 ml of pyridine), the mixture is reacted with 4 eq of N,N' (disuccinimidyl) carbonate (40 mg, 0.158 mmol) at pH 8-9 by adding TEA. The system is left under stirring for 18 hours under argon atmosphere at room temperature. The product is then precipitated with Et₂O in an ice-bath, filtered through a Gooch 3G crucible, washed with Et₂O and recrystallised from EtOH.

**Table 5: ¹H-NMR analysis data of Z-NH-PEG₆₀₀₀-NH₂ (in DMSO)**

| | | **ppm** | **Integral value calc.** | **Integral value found** |
|---|---|---|---|---|
| A | NH₃⁺ | 7.7 | 3 | 2.8 |
| B | H (Z) + NH urethane | 7.4 | 6 | 5.1 |
| C | NH urethane | 7.1 | 2 | 1.4 |
| D | CH₂-Z | 5.0 | 2 | 1.9 |
| E | PEG-CH₂-O-linker | 4.0 | 4 | 4.0 |
| F | PEG (M.W. = 6000) | 3.8-3.2 | 545 | 588 |
| G | -CH₂-NH-CO-O- | 2.9-3.1 | 6 | 5.8 |
| H | -CH₂-NH₃⁺ | 2.8 | 2 | 1.9 |
| I | PEG-NH-CH₂-CH₂-CH₂-NH₃⁺ | 1.7 | 2 | 2.0 |
| L | PEG-NH-CH₂-CH₂-CH₂-NH-Z | 1.5 | 2 | 1.7 |

### 4.b) Synthesis of Z-NH-CO-O-PEG₆₀₀₀-NH-CO-NH-Gly-Phe-COOMe

194 mg (0.03 mmol) of Z-NH-CO-O-PEG-NH-OSu are coevaporated with anhydrous CH₂Cl₂ (x 2) and left to dry using a rotary pump. The PEG-derivative is dissolved in the minimum quantity of anhydrous CH₂Cl₂; 42µl of triethylamine (TEA) are added to the solution thus obtained and the pH adjusted to 8-9. The system is maintained under stirring , adding 10 eq of CF₃COO⁻H₃N⁺-Gly-Phe-COOMe (105 mg, 0.3 mmol). The system is left under stirring for 18 hours at room temperature. The product is then precipitated from Et₂O in an ice-bath, filtered through a Gooch 3G crucible, washed with Et₂O and recrystallized from EtOH.

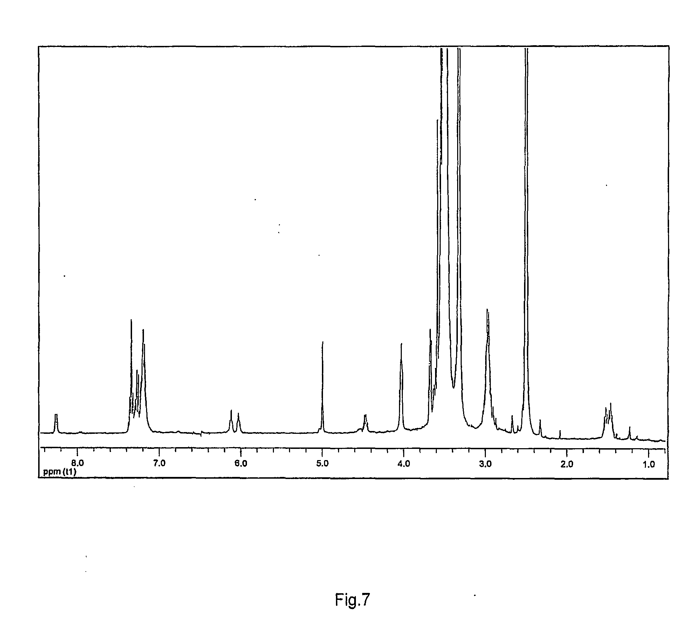

The ¹H-NMR spectrum of the compound is given in Fig. 7/Tab. 6.

**Table 6: ¹H-NMR analysis data of Z-NH-PEG₆₀₀₀-NH-CO-NH-Gly-Phe-COOMe (in DMSO)**

| | | **ppm** | **lntegral value calc.** | **Integral value found** |
|---|---|---|---|---|
| A | -NH-CO- | 8.2 | 1 | 0.9 |
| B | H(Z+Phe) NH urethane | 7.5-7.0 | 13 | 13.1 |
| C | -NH-CO-NH- | 6.2-5.9 | 2 | 1.8 |
| D | CH₂-Z | 5.0 | 2 | 1.8 |
| E | CHα Phe | 4.5 | 1 | 0.8 |
| F | PEG-CH₂-O-linker | 4.0 | 4 | 4.0 |
| G | PEG (M.W. = 6000) | 3.8-3.2 | 545 | 589 |
| H | -CH₂-NH-CO-O- + -CH₂-NH-CO-NH- | 2.9 | 8 | 8.5 |
| L | PEG-NH-CH₂-CH₂-CH₂-NH- | 1.5 | 4 | 3.8 |

### 4.c) Removal of Z; synthesis of H₂N-PEG-NH-CO-NH-Gly-Phe-COOMe

In a 50 ml 3-neck flask, 120 mg of PEG derivative are dissolved in MeOH to which is then added Pd(OH)₂ as catalyst in a quantity equal to 20% by weight on the substrate. The system is left under stirring overnight under H₂ atmosphere at room temperature.

At the end of the reaction, after removal of the catalyst by filtration through a pleated filter, the MeOH is removed under reduced pressure; the solid obtained is re-dissolved in AcCN and then precipitated from Et₂O in an ice-bath, filtered through a Gooch 3G crucible, washed with Et₂O and stored over KOH.

### 4.d) Synthesis of OSu-NH-PEG₆₀₀₀-NH-CO-NH--Gly-Phe-COOMe

Activation via N,N'-(disuccinimidyl) carbonate, is achieved as described in 4.b).

### 4.e) Synthesis of MeCOO-Phe-Val-NH-CO-NH-PEG₆₀₀₀-NH-CO-NH-Phe-Gly-COOMe

100 mg of OSu-NH-PEG-NH-CO-NH-Gly-Phe-COOMe (0.015 mmol) are coevaporated with anhydrous CH₂Cl₂ (x 2) and left to dry using a rotary pump. The PEG derivative is dissolved in the minimum quantity of anhydrous CH₂Cl₂; triethylamine (TEA) is added to the solution thus obtained and the pH adjusted to 8-9. The system is maintained under stirring , adding 10 eq. of CF₃COO⁻H₃N⁺-Val-Phe-COOMe (59.4 mg, 0.15 mmol). The system is left under stirring for 18 hours at room temperature. The product is then precipitated from Et₂O in an ice-bath, filtered through a Gooch 3G crucible, washed with Et₂O and recrystallised from EtOH.

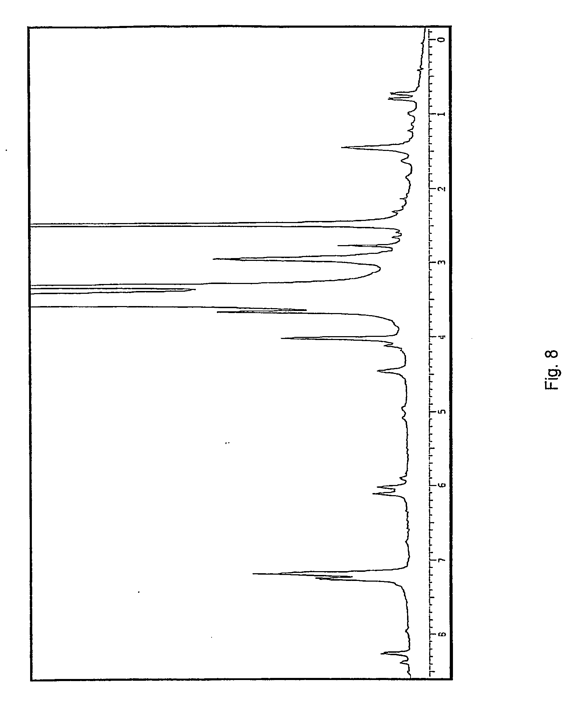

The ¹H-NMR spectrum of the compound is given in fig. 8/tab. 7.

**Table 7: ¹H-NMR analysis data of MeOOC-Phe-Val-NH-CO-O-PEG₆₀₀₀-NH-CO-NH-Gly-Phe-COOMe (in DMSO)**

| | | **ppm** | **Integral value calc.** | **Integral value found** |
|---|---|---|---|---|
| A | -NH-CO-(Val-Phe) | 8.4 | 1 | 0.3 |
| B | -NH-CO-(Gly-Phe) | 8.2 | 1 | 0.9 |
| C | H (Z + 2 Phe) NH urethane | 7.5-7.0 | 17 | 9.9 |
| D | -NH-CO-NH- (Gly-Phe) | 6.2-5.9 | 2 | 1.6 |
| E | -NH-CO-NH- (Phe-Val) | 5.9 | 2 | 0.5 |
| F | 2 CHα Phe | 4.5 | 2 | 1.3 |
| G | PEG-CH₂-O-linker + CHα Val | 4.0 | 5 | 4.4 |
| H | PEG (M.W. = 6000) | 3.8-3.2 | 545 | 588 |
| I | -CH₂-NH-CO-O- + -CH₂-NH-CO-NH- | 2.9 | 8 | 7.9 |
| L | PEG-NH-CH₂-CH₂-CH₂-NH- | 1.5 | 4 | 3.0 |
| M | 2CH₃-(Val) | 0.8 | 6 | 1.4 |

The availability of these biocompatible polymer supports is of great importance mainly in the field of conjugating and carrying both low and high molecular weight drugs. The possibility of introducing chemically different molecules, with different times and manners, onto the same PEG paves the way to a new and wide ranging class of possible drugs with complementary and possibly synergistic activities. To be emphasised is that the availability of said reactive bifunctional PEGs is normally limited to chains of small dimensions, thus having poor functional characteristics for the purposes of the pharmacological properties of the relative conjugates, whereas with the described process PEG derivatives can be obtained starting from polydisperse commercial PEG of any molecular weight including high molecular weight PEG. With the aims of obtaining conjugates between said PEG derivatives and biologically active compounds commercial dihydroxy PEGs of high molecular weight are preferred.

## Claims

1. Bifunctional polyethylene glycol (PEG) derivatives of general formula (I)
P'HN-(H₂C)_{n'}-HN-CO-O-PEG-O-CO-NH-(CH₂)ₙ-NHP (I)
in which:
the polyethylene glycol has a molecular weight between 2,000 and 40,000,
P, P' can be the same or different one from the other and be a hydrogen or a protecting group for the amino group,
n and n', being the same or different one from the other, are between 1 and 6.

2. Bifunctional polyethylene glycol derivatives as claimed in claim 1 wherein P, P', when they are protecting groups for the amino group, are chosen from the group comprising carbamates and amides.

3. Process for synthesising bifunctional polyethylene glycol (PEG) derivatives claimed in claim 1 **characterised by** the following steps:
- derivatization of a first terminal hydroxyl group of the polyethylene glycol with a bifunctional compound activated at one functional group and optionally mono-protected at the second functional group,
- derivatization of the second hydroxyl group of the polyethylene glycol with a bifunctional compound being the same or different from the preceding,
- ionisation of the free functional group of the bifunctional PEG derivative obtained,
- purification of the ionic bifunctional PEG derivative obtained,
- neutralization of the ionised functional group of the purified ionic bifunctional PEG derivative and its optional protection.

4. Use of bifunctional polyethylene glycol (PEG) derivatives as claimed in claim 1 for preparation of conjugates by conjugation with compounds of different kind, being the same or different one from the other, usable for diagnostic, prophylactic and therapeutic purposes or for supported synthesis processes.

5. Use of bifunctional polyethylene glycol (PEG) derivatives claimed in claim 1 as carrier and/or stabilizer of molecules, being the same or different one from the other, by conjugation of said molecules to said bifunctional polyethylene glycol derivatives.

6. Use as claimed in claim 5 wherein said molecules are compounds suitable for diagnostic, prophylactic and therapeutic purposes.

7. Use as claimed in claim 5 wherein said molecules are reagent or catalyst for supported organic synthesis processes.

## Patentansprüche

1. Bifunktionale Polyethylenglykol- (PEG-) Derivate der allgemeinen Formel (I)
P'HN-(H₂C)ₙ,-HN-CO-O-PEG-O-CO-NH-(CH₂)ₙ-NHP (I)
in der:
das Polyethylenglykol ein Molekulargewicht zwischen 2.000 und 40.000 hat, P, P' gleich oder voneinander verschieden sein können und ein Wasserstoff oder eine Schutzgruppe für die Aminogruppe sein können, n und n', die gleich oder voneinander verschieden sind, zwischen 1 und 6 liegen.

2. Bifunktionale Polyethylenglykolderivate gemäß Anspruch 1, wobei P, P', wenn sie Schutzgruppen für die Aminogruppe sind, aus der Carbamate und Amide umfassenden Gruppe ausgewählt werden.

3. Verfahren zur Herstellung von bifunktionalen Polyethylenglykol- (PEG-) Derivaten gemäß Anspruch 1, **gekennzeichnet durch** die folgenden Schritte:
- Derivatisierung einer ersten terminalen Hydroxylgruppe des Polyethylenglykols mit einer bifunktionalen Verbindung, die an einer funktionalen Gruppe aktiviert ist und gegebenenfalls an der zweiten funktionalen Gruppe einfach geschützt ist,
- Derivatisierung der zweiten Hydroxylgruppe des Polyethylenglykols mit einer bifunktionalen Verbindung, die gleich der vorhergehenden oder von ihr verschieden ist,
- Ionisierung der freien funktionalen Gruppe des erhaltenen bifunktionalen PEG-Derivats,
- Reinigung des erhaltenen ionischen bifunktionalen PEG-Derivats,
- Neutralisierung der ionisierten funktionalen Gruppe des gereinigten, ionischen, bifunktionalen PEG-Derivats und sein optionaler Schutz.

4. Verwendung von bifunktionalen Polyethylenglykol- (PEG-) Derivaten gemäß Anspruch 1 für die Herstellung von Konjugaten durch Konjugation mit Verbindungen von unterschiedlicher Art, welche die gleichen oder voneinander verschieden sind, die für diagnostische, vorbeugende und therapeutische Zwecke oder für trägergestützte Herstellungsverfahren verwendet werden können.

5. Verwendung von bifunktionalen Polyethylenglykol- (PEG-) Derivaten gemäß Anspruch 1 als Träger und/oder Stabilisierungsmittel von Molekülen, die gleich oder voneinander verschieden sind, durch Konjugation der genannten Moleküle an genannte bifunktionale Polyethylenglykolderivate.

6. Verwendung gemäß Anspruch 5, wobei genannte Moleküle Verbindungen sind, die für diagnostische, vorbeugende und therapeutische Zwecke geeignet sind.

7. Verwendung gemäß Anspruch 5, wobei genannte Moleküle Reagenzien oder Katalysatoren für trägergestützte organische Syntheseverfahren sind.

## Revendications

1. Dérivés bifonctionnels de polyéthylèneglycol (PEG) de formule générale (I)
P'HN-(H₂C)_{n'}-HN-CO-O-PEG-O-CO-NH-(CH₂)ₙ-NHP (I)
dans lesquels :
le polyéthylèneglycol présente une masse moléculaire comprise entre 2 000 et 40 000,
P, P' peuvent être identiques ou différents l'un de l'autre et être un hydrogène ou un groupe protecteur pour le groupe amino,
n et n', étant identiques ou différents l'un de l'autre, sont compris entre 1 et 6.

2. Dérivés bifonctionnels de polyéthylèneglycol selon la revendication 1, dans lesquels P, P', lorsque ce sont des groupes protecteurs pour le groupe amino, sont choisis dans le groupe constitué de carbamates et d'amides.

3. Procédé de synthèse des dérivés bifonctionnels de polyéthylèneglycol (PEG) selon la revendication 1, **caractérisé par** les étapes suivantes :
- dérivation d'un premier groupe hydroxyle terminal du polyéthylèneglycol avec un composé bifonctionnel activé au niveau d'un groupe fonctionnel et facultativement mono protégé au niveau du deuxième groupe fonctionnel,
- dérivation du deuxième groupe hydroxyle du polyéthylèneglycol avec un composé bifonctionnel identique ou différent du précédent,
- ionisation du groupe fonctionnel libre du dérivé bifonctionnel PEG obtenu,
- purification du dérivé bifonctionnel PEG ionique obtenu,
- neutralisation du groupe fonctionnel ionisé du dérivé bifonctionnel PEG ionique purifié et sa protection facultative.

4. Utilisation de dérivés bifonctionnels de polyéthylèneglycol (PEG) selon la revendication 1 pour la préparation de conjugués par conjugaison avec des composés de type différent, identiques ou différents les uns des autres, utilisables à des fins diagnostiques, prophylactiques et thérapeutiques ou pour des procédés de synthèse pris en charge.

5. Utilisation de dérivés bifonctionnels de polyéthylèneglycol (PEG) selon la revendication 1 comme support et/ou stabilisateur de molécules, identiques ou différentes les unes des autres, par conjugaison desdites molécules avec lesdits dérivés bifonctionnels de polyéthylèneglycol.

6. Utilisation selon la revendication 5, dans laquelle lesdites molécules sont des composés convenant à des fins diagnostiques, prophylactiques et thérapeutiques.

7. Utilisation selon la revendication 5, dans laquelle lesdites molécules sont un réactif ou un catalyseur pour des procédés de synthèse organique pris en charge.
